(19) **Europäisches Patentamt European Patent Office Office européen des brevets**

(11) **EP 0 737 246 B1**

(12) **FASCICULE DE BREVET EUROPEEN**

(45) Date de publication et mention
de la délivrance du brevet:
**17.12.2008 Bulletin 2008/51**

(51) Int Cl.:
*C12N 1/20* (2006.01)      *A23L 1/00* (2006.01)
*C12P 7/06* (2006.01)      *C12P 7/40* (2006.01)
*C02F 3/34* (2006.01)      *C12R 1/225* (2006.01)
*C12R 1/07* (2006.01)

(21) Numéro de dépôt: 95907047.5

(22) Date de dépôt: **18.01.1995**

(86) Numéro de dépôt international:
**PCT/FR1995/000056**

(87) Numéro de publication internationale:
**WO 1995/019425 (20.07.1995 Gazette 1995/31)**

(54) **SOUCHES BACTERIENNES DU GENRE BACILLUS, PHENOTYPIQUEMENT PROCHES DU GENRE LACTOBACILLUS, PROCEDE DE CULTURE ET APPLICATIONS**

BAKTERIENSTAEMME DES GENUS BACILLUS, PHENOTYPISCH LACTOBACILLUS-AEHNLICH, VERFAHREN ZUR KULTIVIERUNG UND VERWENDUNG

BACTERIAL STRAINS OF GENUS BACILLUS CLOSELY PHENOTYPICALLY RELATED TO GENUS LACTOBACILLUS, CULTURE METHOD AND USE

(84) Etats contractants désignés:
**AT BE CH DE DK ES FR GB GR IE IT LI LU MC NL PT SE**

(30) Priorité: **18.01.1994  FR 9400485**

(43) Date de publication de la demande:
**16.10.1996  Bulletin 1996/42**

(73) Titulaire: **Institut de Recherche pour le Développement ( IRD)
75010 Paris (FR)**

(72) Inventeurs:
• **COMBET-BLANC, Yannick**
  **F-13005 Marseille (FR)**
• **OLLIVIER, Bernard**
  **F-13360 Roquevaire (FR)**
• **GARCIA, Jean-Louis**
  **F-13090 Aix-en-Provence (FR)**

(74) Mandataire: **Peaucelle, Chantal et al
Cabinet Armengaud Aîné
3, Avenue Bugeaud
75116 Paris (FR)**

(56) Documents cités:
**EP-A- 0 113 215          EP-A- 0 298 641**

• **OKAFOR N: "Microbiology of Nigerian Palm Wine with Particular Reference to Bacteria" JOURNAL OF APPLIED BACTERIOLOGY, vol. 38, 1975, pages 81-88, XP008073909**

EP 0 737 246 B1

EP 0 737 246 B1

**Description**

L'invention a pour objet des souches bactériennes phénotypiquement proches du genre Lactobacillus.

Elle vise également des procédés de culture de ces souches ainsi que leurs applications industrielles.

L'invention se rapporte plus particulièrement à des souches bactériennes telles qu'isolées de vin de palme.

On sait que le vin de palme est une boisson alcoolique provenant de la fermentation spontanée de la sève d'arbres appartenant à la famille des palmiers. Cette sève contient environ 10 à 15 % (p/v) de saccharose, de nombreux acides aminés et vitamines.

Les études microbiologiques effectuées ont montré la présence d'une abondante flore mésophile constituée par de nombreuses espèces de bactéries et levures.

Ainsi, dans J.appl. Bact., 1975, 28, 81-88, M. Okafor décrit des bactéries isolées d'échantillons de vin de palme.

L'étude par les inventeurs de la flore anaérobie thermophile présente dans un vin de palme provenant du Sénégal les a conduits à isoler une souche non identifiée à ce jour présentant des propriétés de grand intérêt dans divers domaines de l'industrie.

L'invention a donc pour but de fournir de nouvelles souches de ce type.

Elle vise également à fournir des protocoles de culture de ces souches précisant les conditions physico-chimiques et la composition du milieu de culture qui permettent de produire favorablement des cellules et/ou certains métabolites comme par exemple du L(+) lactate.

Selon un autre aspect, l'invention vise l'utilisation directe de ces souches ou celle de leurs métabolites dans diverses industries.

L'invention vise en particulier la souche bactérienne déposée à la C.N.C.M. le 24 novembre 1993 sous le n° I 1378 et les souches de la même espèce possédant une catalase, ayant une température de croissance de 25°C à 58,5°C avec une température de croissance optimale de 47°C à 53°C, possédant des propriétés amylolytiques et/ou capables de produire du L(+) lactate en tant que produit de fermentation.

La référence d'identification de cette souche est DKP. Comme nom de désignation taxonomique, on utilisera Bacillus thermoamylovorans gen. nov. sp. nov, la désignation préalable étant Lactobacter thermoamylovorans gen. nov. sp. nov.

De manière avantageuse, au moins 70 % du génome des souches de l'invention est capable de s'hybrider avec l'ADN de la souche déposée.

Selon un autre aspect, ces souches sont caractérisées en ce qu'elles sont modérément thermophiles, possèdent des propriétés amylolytiques et/ou sont capables de produire du lactate à partir d'hydrates de carbone. On notera que, de manière avantageuse le lactate produit est à plus de 95 % du L(+) lactate.

Par l'expression "modérément thermophile", on entend des souches bactériennes capables de croître à des températures de l'ordre de 25 à 58,5°C, avec un optimum se situant entre 47 et 53°C.

L'invention vise plus particulièrement des souches du genre Bacillus tel que montré par comparaison des séquences de l'ARN de la fraction 16 S des ribosomes.

Des souches de ce type sont encore caractérisées en ce qu'elles sont hétérolactiques, c'est-à-dire qu'elles sont capables, à partir d'hydrates de carbone, de produire des composés autres que le L(+) lactate, notamment de l'éthanol, du formate et de l'acétate, selon les conditions physico-chimiques du milieu de culture, en particulier selon le pH, et le rapport sucres/peptides.

De telles souches sont encore caractérisées en ce qu'elles ne possèdent pas de cytochrome et ne sont pas capables d'utiliser les nitrates ou les sulfates comme accepteur terminal d'électrons. Elles possèdent une catalase et leur métabolisme est anaérobie facultatif.

Selon une autre disposition de l'invention, ces souches sont caractérisées par des conditions de croissance anaérobies et des propriétés de microaérophilie, c'est-à-dire que de faibles pressions partielles d'oxygène stimulent leur croissance.

Leur croissance s'effectue plus spécialement à un pH de 5,4 à 8,5, avec un optimum pour la croissance de 6,5 à 7,5 environ.

Les souches bactériennes de l'invention sont encore **caractérisées en ce qu'**elles sont Gram positif.

Selon une autre disposition de l'invention, les souches bactériennes sont caractérisées par des cellules non sporulées, isolées ou en chaînettes de 3 ou 4 cellules, ayant la forme de bâtonnets avec des flagelles péritriches.

Selon encore une autre disposition, la teneur de l'ADN des souches bactériennes de l'invention en guanine plus cytosine est inférieure à 50 mole %, en particulier de l'ordre de 40 mole %.

Les mutants des souches répondant aux définitions qui précèdent entrent également dans le cadre de l'invention dès lors qu'ils conservent au moins 70 % de capacité d'hybridation avec l'ADN de la souche déposée.

Conformément à l'invention, les souches bactériennes définies ci-dessus sont obtenues par culture dans des conditions anaérobies, ou tout au moins sous faible pression partielle d'oxygène, à un pH de 5,4 à 8,5 environ, dans un milieu de base contenant un sucre utilisable par ces souches comme source d'énergie et des peptides comme source d'azote, à une température de 25 à 58,5°C.

Par culture sous faible pression partielle d'oxygène, on entend une culture effectuée par exemple en milieu liquide avec de l'air au-dessus.

On effectue avantageusement la culture à un pH de 6,8 à 7,2 et à une température de 47 à 53°C.

Dans des conditions optimales de pH et de température, le temps de doublement de la biomasse est de 40 à 45 minutes environ.

Pour la croissance des souches, on utilise principalement des hydrates de carbone, plus particulièrement du glucose, comme composés source de carbone et d'énergie, des peptides comme source d'azote et facteurs de croissance.

Les peptides et les facteurs de croissance sont présents par exemple dans des extraits de levure et des hydrclysats trypsiques de caséine ou de gélatine.

L'addition de vitamines permet d'accélérer la croissance mais n'est pas indispensable.

Celle-ci est particulièrement favorisée lorsqu'on ajoute au milieu de culture un hydrolysat de caséine tel qu'obtenu par action de la trypsine, comme celui appelé couramment Biotryptase[R] ou des extraits de levure.

Les colonies bactériennes formées après environ 48 h de culture dans les conditions données ci-dessus sont reprises isolément en milieu liquide afin d'obtenir des cultures pures.

Différentes propriétés des souches de l'invention ont été indiquées dans la description qui précède. Ces propriétés trouvent des applications dans des domaines très variés.

Ainsi ces souches, grâce à leurs propriétés thermophiles et amylolytiques, qui s'exercent également en milieux très dilués, sont avantageusement ajoutées aux effluents industriels à des fins de dépollution.

L'invention vise donc un procédé de traitement d'effluents industriels à des fins de dépollution, caractérisé en ce qu'il comprend à mise en ouvre de souches bactériennes telles que définies ci-dessus.

La proximité phylogénétique des souches de l'invention avec les espèces appartenant au genre Bacillus ainsi que les similitudes physiologiques avec les bactéries lactiques, sont avantageusement mises à profit dans des procédés de fermentation alimentaire. Leurs propriétés fermentaires et enzymatiques permettent d'y remplacer avantageusement et/ou de compléter celles attribuées aux bactéries lactiques et aux Bacilli.

Ces souches présentent également un grand intérêt pour élaborer des mutants par transfert de matériel génétique entre ces souches, les bactéries lactiques et les bacilli, ce qui permet de cumuler un ensemble de propriétés communes à ces bactéries.

Les caractères thermophile modéré, anaérobie facultatif et neutrophile des souches de l'invention présentent également un grand intérêt en vue d'une exploitation en culture pure. En effet, la possibilité de les cultiver à une température allant jusqu'à 58,5°C permet de limiter les sources de contamination par d'autres microorganismes, d'accélérer les cinétiques du métabolisme cellulaire et d'augmenter la solubilité des composants du milieu de culture. De plus, à cette température la faible concentration d'oxygène dissous n'inhibe pas, mais stimule, la croissance de ces souches anaérobies facultatives, ce qui facilite grandement la mise en oeuvre des cultures en évitant toutes les contraintes techniques liées à l'anaérobiose. Le caractère neutrophile des souches, appréciable par rapport à l'acidophilie des bactéries lactiques, permet d'éviter les problèmes de corrosions et d'allonger la durée de vie des installations.

La capacité des souches de l'invention à fermenter une grande variété de sucres, notamment l'amidon, le lactose et le saccharose est un atout important. Ces trois sucres potentiellement utilisables comme substrats énergétiques sont en effet, disponibles sous différentes formes en grande quantité.

La possibilité d'agir sur le métabolisme de ces souches en contrôlant les paramètres physico-chimiques du milieu de culture (pH, rapport sucres/peptides) élargit leur domaine d'application. Ainsi, il est possible par exemple d'orienter la fermentation vers la production de cellules et de métabolites cellulaires tels que des enzymes. Il est également possible d'orienter le métabolisme énergétique de la souche en favorisant soit la production de lactate, soit la production d'acétate, d'éthanol et de formate.

L'invention vise donc également un procédé de production de métabolites tels que le L(+) lactate, l'acétate, le formate, l'éthanol, caractérisé en ce qu'il comprend

- la culture d'une souche bactérienne telle que définie ci-dessus, dans des conditions appropriées pour son développement et pour la production du métabolite recherché,
- la récupération des métabolites produits, suivi de l'isolement du métabolite désiré et sa purification.

L'acide lactique produit par les souches de l'invention est d'un grand intérêt car il est constitué à plus de 95 % par du L(+) lactate qui est assimilable par les organismes supérieurs alors que le D(-) lactate présente un caractère de toxicité.

On le sépare de la culture, et on le concentre par exemple par évaporation, le cas échéant jusqu'à siccité.

Les concentrés ou produits secs sont utilisés tels quels ou traités pour former des dérivés souhaités de l'acide lactique.

On sait que les applications de l'acide lactique ou de ses esters et autres dérivés sont très larges.

L'acide lactique est ainsi utilisé dans l'industrie agro-alimentaire en l'incorporant dans des boissons, des bières, des produits laitiers tels que crème, fromage, beurre, des glaces ou encore des confitures.

Comme tensio-actif, on l'utilisera avec avantage en panification et viennoiserie sous forme par exemple de lactyl mono- et diglycérides et de sodium stéaryl lactylate.

Dans l'industrie pharmaceutique le lactate de potassium peut constituer un substitut du chlorure de sodium particulièrement précieux dans les cas d'hypertension.

Il est aussi utilisé pour ses propriétés de complexant, notamment avec le fer et le calcium pour traiter les carences.

Enfin parmi les applications de l'acide lactique, de ses sels et dérivés dans l'industrie chimique, on citera son utilisation dans l'élaboration de résines plastiques, d'adhésifs, de pesticides, de textiles, ou encore dans des peintures, des diluants et des solvants, ou pour le traitement de surface de métaux.

On soulignera son grand intérêt dans la chimie des polymères où il sert à fabriquer des polylactides et/ou des copolymères avec par exemple des oxydes de polyalkylène, des alcools polyvalents, de l'acide glycolique, des acides hydroxycarboxyliques, des copolymères d'éthylène et de propylène, des caoutchouc butyliques ou des élastomères de polyuréthane thermoplastiques. A partir de ces polymères et/ou copolymères, divers articles peuvent être fabriqués en particulier pour l'emballage, des films à usages médicaux pour réaliser des pansements ou encore des matières d'enrobage pour sutures chirurgicales.

Le milieu de culture préconisé pour l'utilisation industrielle des souches de l'invention en vue de la production de L(+)lactate présente l'intérêt d'être à un coût inférieur à celui habituellement utilisé pour les bactéries lactiques, eu égard aux prix de l'extrait de levure ou du "corn steep liquor" contenant les facteurs de croissance indispensables à ces bactéries. En effet, les bactéries lactiques étant très exigentes en facteurs de croissance, il est recommandé pour une bonne productivité en acide lactique d'utiliser un milieu dont le rapport sucre/facteurs de croissance est de 1 alors que pour les souches de l'invention ce rapport doit être de 2.

D'autres caractéristiques et avantages de l'invention sont rapportés dans la description qui suit donnée à titre d'exemple, qui concerne la souche mentionnée plus haut, dite souche DKP, déposée à la C.N.C.M. sous le n° I-1378.

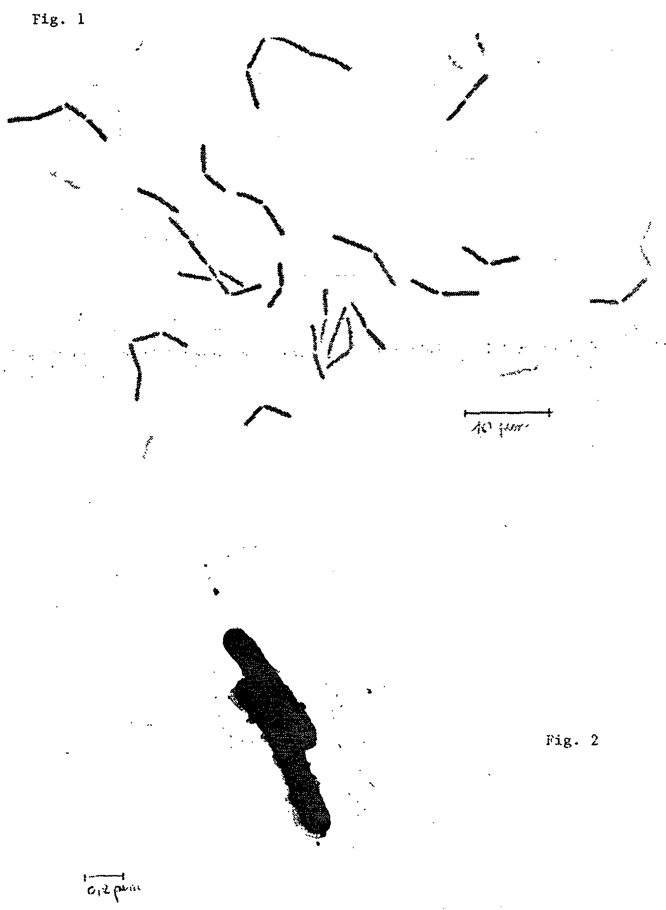

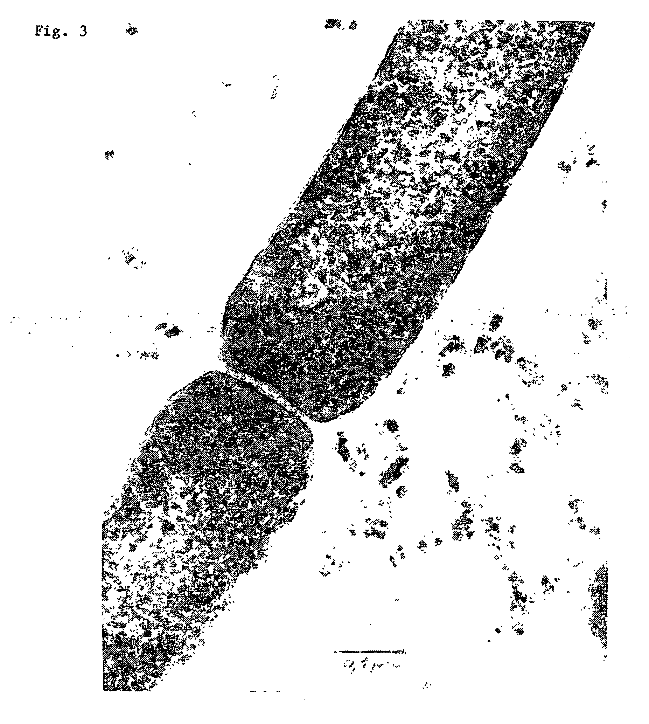

Il y sera fait référence aux figures 1 à 3 qui représentent des photos de cellules de cette bactérie au microscope optique (figure 1) et au microscope électronique , avec un grossissement de 20 000 sur la figure 2 et de 80 000 sur la figure 3.

## a. PROCEDURE D'ENRICHISSEMENT ET ISOLEMENT DES SOUCHES

. Milieux et méthodes de culture

On utilise le milieu de culture suivant :

| | |
|---|---|
| Glucose | 10 g |
| Extrait de levure | 5 g |
| Biotrypcase | 5r g |
| $KH_2PO_4$ | 1 g |
| $MgCl_2, 6H_2O$ | 0,4 g |
| $NH_4Cl$ | 1 g |
| $FeSO_4, 7H_2O$ | 5 mg |
| Solution minérale de Balch (ci-dessous) | 25 ml |
| Solution d'oligoéléments de Balch (ci-dessous) | 1 ml |
| Tween 80 | 1 ml |
| $H_2O$ distillée q.s.p. | 1000 ml |

Les compositions de la solution minérale de Balch et de la solution d'oligoéléments de Balch sont les suivantes :

| Solution minérale de Balch | |
|---|---|
| $KH_2PO_4$ | 6 g |
| $(NH_4)_2SO_4$ | 6 g |
| NaCl | 12 g |
| $MgSO_4,7H_2O$ | 2,6 g |

(suite)

| | |
|---|---|
| $CaCl_2, 2H_2O$ | 0,16 g |
| $H_2O$ distillée q.s.p. | 1000 ml |

**Solution d'oligo-éléments de Balch**

| | |
|---|---|
| Acide nitriloacétique | 1,5 g |
| $MnSO_4, 2H_2O$ | 0,5 g |
| $MgSO_4, 7H_2O$ | 3 g |
| NaCl | 1 g |
| $FeSO_4, 7H_2O$ | 0,1 g |
| $CoCl_2, 6H_2O$ | 0,1 g |
| $CaCl_2, 2H_2O$ | 0,1 g |
| $ZnCl_2$ | 0,1 g |
| $CuSO_4, 5H_2O$ | 0,01 g |
| $AlK(SO_4)_2$ | 0.01 g |
| $H_3BO_3$ | 0,01 g |
| $Na_2MoO_4$ | 0,01 g |
| $H_2O$ distillée q.s.p. | 1000 ml |

Le pH du milieu de culture est ajusté à pH 7,5 avec KOH 10 M avant stérilisation.

Le milieu est ensuite porté à ébullition, puis refroidi et réparti sous un courant d'azote

- dans des flacons de type pénicilline ou flacons sérum à raison de 20 ml par flacon
- dans des tubes de Hungate, à raison de 10 ml par tube, et
- dans des tubes de Hungate renfermant 4,5 ml de milieu à 3,5 % d'agar par tube.

Après traitement à l'autoclave à 110°C pendant 45 min, on ajoute :

- 1 ml de $NaHCO_3$ à 10 % + 0,2 ml de $Na_2S$, $9H_2O$ à 2 % dans les flacons sérum,
- 0,5 ml de $NaHCO_3$ à 10 % + 0,1 ml de $Na_2S$, $9H_2O$ à 2 % dans les tubes de Hungate à 10 ml, et
- 0,25 ml de $NaHCO_3$ à 10 % + 0,05 ml de $Na_2S$, $9H_2O$ à 2 % dans les tubes de Hungate contenant de l'agar.

Les solutions de $NaHCO_3$ et de $Na_2S$ utilisées sont stériles.

Les milieux, liquides dans les deux premiers cas, solide dans le dernier cas, sont prêts à être inoculés.

. Procédure d'enrichissement :

On soumet à incubation à une température de 50°C des flacons contenant du milieu de culture préparé anaérobiquement comme défini plus haut et inoculés avec des échantillons de vin de palme. Après plusieurs repiquages, la flore anaérobie et thermophile initialement présente dans le vin de palme est devenue dominante.

. Procédure d'isolement par la technique des rolls tubes selon la méthode de Hungate et Macy (1) et (2) :

- préparation des rolls tubes.

Les tubes de Hungate sont chauffés à 90°C aux fins de fusion du milieu solide. Ils sont ensuite refroidis à 50°C avec maintien de l'agar en fusion.

On inocule avec le milieu d'enrichissement final tel qu'obtenu ci-dessus à raison de 0,5 ml dans chaque tube.

La gélose en fusion est répartie, par centrifugation, sur la paroi du tube et solidifiée par refroidissement.

- Isolement

Après 48 h d'incubation à 50°C des rolls tubes, chaque colonie est resuspendue et diluée en cascade dans une série de tube de Hungate renfermant un milieu liquide.

Les tubes sont incubés à 50°C pendant 24 heures.

On inocule ensuite un flacon sérum contenant du milieu liquide à partir de la dernière dilution montrant une croissance et on soumet le flacon à incubation à 50°C pendant 24 heures.

On vérifie au microscope optique que toutes les cellules sont identiques.

La procédure d'isolement est répétée au moins une fois en vérifiant que toutes les colonies présentent des caractéristiques identiques.

La culture résultante est pure.

**b. Description de la souche**

. Caractères morphologiques

Après 48 heures d'incubation des rolls tubes, la souche obtenue appelée souche DKP se présente sous forme de colonies blanches et lisses avec un diamètre de 1 à 1,5 mm. Les cellules non sporulées, isolées ou en chaînette de 3 à 4 cellules (voir photo 1), ont la forme de bâtonnets de 0,35 à 0,4 $\mu$m de diamètre et de 3 à 4 $\mu$m de longueur possédant des flagelles péritriches (voir photo 2 ). La paroi cellulaire présente les caractéristiques des bactéries Gram positives (voir photo 3).

. Caractères biochimiques et propriétés métaboliques.

- Eléments du milieu

Dans un milieu contenant des hydrates de carbone, notamment du glucose comme source d'énergie, on ajoutera avec avantage des peptides, par exemple des extraits de levure, pour favoriser la croissance. Aucune croissance n'est observée lorsqu'on utilise de la gélatine ou de la caséine à la place de l'extrait de levure. Mais on constate une faible croissance lorsqu'on ajoute des acides aminés à la place de l'extrait de levure.

- Conditions de croissance et propriétés métaboliques

La croissance s'effectue dans des conditions anaérobies ou tout au moins sous une faible pression partielle d'oxygène. Les colonies ne sont pas toujours observées sur des boites de Pétri incubées en aérobiose. Les cellules possèdent une catalase, mais pas de cytochromes. $NO_3$- et $SO_4$= ne sont pas réduits et on n'observe aucune production d'indole, de $H_2S$ et de $H_2$. Dans un milieu avec de l'arginine comme source d'énergie, $NH_3$ est produit mais aucune croissance n'est observée.

Les valeurs de pH pour la croissance sont de 5,4 à 8,5 avec un optimum à 7,0.

La température optimale pour la croissance se situe autour de 50°C, 58,5°C étant la température limite supérieure.

Dans un milieu contenant de l'extrait de levure et du glucose comme sources d'énergie, à pH de 7,0 et à une température de 50°C, le temps de doublement maximum de la biomasse est de 40 à 45 min.

Les produits de la fermentation du glucose sont l'acétate, l'éthanol, le formate et le lactate. Le lactate produit est à 96 % du L(+) lactate. Les proportions molaires en acétate, éthanol et formate sont de l'ordre respectivement de 1:1:2 alors que la proportion du lactate par rapport à ces trois produits varie selon les conditions de culture (pH et rapport glucose/peptides).

- Sucres fermentescibles

Les sucres suivants sont fermentés en 24 heures : L-arabinose, ribose, D-glucose, D-fructose, D-mannose, rhamnose, amygdaline, arbutine, esculine, salicine, cellobiose, maltose, trehalose, amidon, glycogène, β-gentiobiose et gluconate.

Une fermentation plus lente de 48 à 96 h est observée avec le D-xylose, le galactose, la N-acétyl-D-glucosamine, le lactose, le saccharose, le melezitose, et le D-turanose.

Aucune fermentation n'est observée pour le glycérol, l'érythritol, le D-arabinose, le L-xylose, l'adonitol, le L-sorbose, le dulcitol, l'inositol, le mannitol, le sorbitol, un méthyl-D-mannoside, un méthyl-D-glucoside, le mélibiose, l'inuline, le D-raffinose, le xylitol, le D-lyxose, le D-tagatose, le D et L-fucose, le D-arabitol et le L-arabitol.

- Besoins en vitamines :

On observe une stimulation de la croissance de la souche bactérienne avec la thiamine, la D, L-biotine, les bases puriques et pyrimidiques. En revanche, aucun effet significatif n'apparaît avec la vitamine B12, la pyridoxine, l'acide nicotinique, l'acide p-aminobenzoïque, le Ca-D-pantothénate, l'acide folique et la riboflavine.

Caractères génétiques :

La souche DKP est caractérisée par une teneur en guanine + cytosine, déterminée par HPLC selon la méthode de Meshbah et al (3), de 38,8 $\pm$ 0,2 %.

## c. TECHNIQUES D'ANALYSE

La croissance bactérienne est contrôlée en mesurant l'augmentation de turbidité à 600 nm dans des tubes de Hungate pour cultures anaérobies avec un spectrophotomètre Shimadzu UV 160A.

Le profil fermentaire est déterminé en utilisant le milieu de base auquel on ajoute stérilement l'hydrate de carbone testé jusqu'à une concentration finale de 0,3 % (p/v) et 0,017 % de bleu de bromothymol. Pour chaque hydrate de carbone, stérilisé séparément par filtration, on effectue deux cultures en double successives.

Les besoins en vitamines sont déterminés en utilisant un milieu chimiquement défini dépourvu de vitamines, supplémenté en glucose jusqu'à une concentration finale de 0,3 % (p/v).

Les vitamines et bases puriques et pyrimidiques sont ajoutées stérilement à une concentration finale : acide nicotinique, thianine HCl, Ca-D- pantothénate, riboflavine, 992 $\mu$g/l ; acide p-aminobenzoïque, 551 $\mu$g/l; pyridoxine, 1984 $\mu$g/l ; vitamine B12, 1 $\mu$g/l ; DL biotine, 10 $\mu$g/l ; acide folique, 1 $\mu$g/l ; adénine, guanine, uracile, xanthine, 5157 $\mu$g/l. Pour les inoculations, on utilise des suspensions de bactéries qui ont été lavées deux fois dans de l'eau physiologique à 9 º/oo de NaCl stérile. Pour chaque vitamine, on effectue trois cultures successives.

Les formes de résistance (spores) ont été recherchées par observation au microscope et par mise en culture de la souche après pasteurisation à 80°C pendant 20 minutes.

L'intervalle de température pour la croissance a été défini dans des cultures en tube incubés dans des bains maries régulés à différentes températures.

L'activité catalase est testée avec une solution à 3 % (v/v) de peroxyde d'hydrogène. La production d'indole et d'ammonium sont déterminées respectivement avec les réactifs de Kovacs et de Nessler. Pour révéler la réduction des nitrates, on utilise le réactif de Griess.

$H_2S$ est mesuré par photométrie en tant que colloïde CuS, après réaction avec un mélange contenant 50 mM de HCl et 5 mM de $CuSO_4$.

Le glucose, le lactate, l'acétate, l'éthanol et le formate sont déterminés sur des échantillons dilués par HPLC en utilisant une pompe Analprep 93 et une colonne de type ORH 801. On utilise un débit de 0,6 ml/min, un volume de boucle d'injection de 20 $\mu$l, une température de colonne de 35°C. Le détecteur utilisé est un réfractomètre différentiel (Knauer, Berlin, RFA).

La quantité d'hydrogène est déterminée à l'aide d'un chromatographe gazeux Girdel série 30, équipé d'un détecteur de conductivité thermique. La colonne est remplie avec du Carbosphere SS (mesh 60/80).

Les déshydrogénases L (+) lactique et D (-) lactique sont utilisées pour déterminer les formes stéréoisomères de l'acide lactique produit par la fermentation du glucose (kits enzymatiques Boehringer Mannheim).

Pour l'analyse des cytochromes, on soumet 3 g de cellules à un traitement de sonication dans 10 ml de tampon 20 mM Tris chlorhydrate (pH 7,6) ; la suspension de cellules cassées est centrifugée à 30000 g pendant 45 min à 5°C pour éliminer les débris cellulaires. Le surnageant résultant est séparé en un surnageant et une fraction particulaire par centrifugation à 140000 g pendant 2 h. Le surnageant est pris en tant que fraction soluble. Le culot gélatineux noir est remis en suspension dans le même tampon et représente la fraction particulaire. L'examen pour les cytochromes des extraits dépourvus de cellules est réalisé à l'aide d'un spectrophotomètre Shimadzu modèle UV 300.

## d. PROCEDES DE CULTURE ET APPLICATIONS

1- PRODUCTION DE BIOMASSE

Pour la production de biomasse, deux techniques de culture sont utilisées :

1- En milieu non renouvelé.

La fermentation est régulée à un pH de 7 à l'aide d'une solution alcaline (soude par exemple) et à une température de 50°C. On utilise un milieu de culture répondant à la composition suivante :

- Glucose à calculer
- Extrait de levure/hydrolysat de protéines à calculer

- NH$_4$Cl       3 g/l
- KH$_2$PO$_4$       3 g/l
- MgCl$_2$,6H$_2$O       0,4 g/l
- FeSO$_4$,7H$_2$O       5 mg/l
- Tween 80       5 g/l

La concentration en glucose et en facteurs de croissance est fonction de la concentration en cellules que l'on souhaite obtenir. Pour estimer la concentration en biomasse en fin de fermentation, la relation suivante peut être utilisée :

$$1/X = 2,07/F + 3,78/G$$

. X concentration cellulaire en g/l (poids sec de cellules par litre)

. G concentration en glucose en g/l

. F Facteurs de croissance contenus dans un mélange constitué à 50 % d'extrait de levure et 50 % d'un hydrolysat de protéine (par exemple caséine hydrolysée par de la trypsine), F étant la concentration en g/l du mélange.

La relation ci-dessus a été vérifiée pour une gamme de concentration en glucose et en facteurs de croissance allant respectivement de 5 à 60 g/l et 1 à 30 g/l.

A titre d'exemple pour obtenir 7,6 g de cellules (poids sec), on utilise 60 g de glucose et 30 g de facteurs de croissance. Dans les conditions opératoires précédemment décrites, la fermentation dure environ 7 à 9 heures, la concentration finale en acide lactique représente environ 40 % en poids des produits finaux et la productivité en cellules est de 1 g/l.h.

2. En milieu renouvelé.

La culture est régulée à un pH de 7 et à une température de 50°C. Le fermenteur est alimenté en continu avec le milieu de culture précédent dont les concentrations en facteurs de croissance (F) et en glucose sont dépendantes de la concentration en cellules que l'on souhaite obtenir. Pour produire de la biomasse, il est recommandé de choisir un taux de dilution de 1 h$^{-1}$ avec comme facteur nutritionnel limitant la croissance, la source d'énergie (glucose ou autres hydrates de carbone). En prenant le glucose comme source d'énergie, il conviendra de choisir un rapport glucose/F d'environ 0,75. Pour calculer les concentrations en facteurs de croissance (F) et en glucose on utilisera les deux demandes spécifiques suivantes.

$$q\ F = 6,6\ g/g.h$$

$$q\ G = 3,5\ g/g.h$$

.q F demande spécifique en facteurs de croissance exprimée en g par g de cellules (poids sec) et par heure.
.q G demande spécifique en glucose exprimée en g de glucose par g de cellules (poids sec).

A titre d'exemple en utilisant un milieu de culture dont les concentrations en glucose et en facteurs de croissance sont respectivement 20 et 40 g/l et en fixant le taux de dilution à 1h-1 (temps de rétention hydraulique de 1 heure), on obtient un jus de fermentation dont la concentration en cellules est de 6 g/l (poids sec) avec une productivité cellulaire de 6 g par litre de fermenteur et par heure. Dans ces conditions le jus de fermentation contient environ 2,5 g/l d'acide lactique, 5,5 g/l d'acide acétique, 4,2 g/l d'éthanol et 8,4 g/l d'acide formique.

2- Production d'acide lactique

En milieu renouvelé.

La culture est régulée à un pH de 6 et à une température de 50°C. Le fermenteur est alimenté en continu avec un milieu de culture dont les concentrations en facteurs de croissance (F) et en glucose sont dépendantes de la concentration en cellules que l'on souhaite obtenir. Pour produire de l'acide lactique, il est recommandé de choisir un taux de dilution de 0,4 h$^{-1}$ comme nutriments limitant les facteurs de croissance F. En prenant le glucose comme source d'énergie, il convient de choisir un rapport glucose/F d'environ 2. Pour calculer les concentrations en facteurs de croissance (F) et en glucose, on utilisera les deux demandes spécifiques suivantes.

$$q\ F = 2,5\ g/g.h$$

$$q\ G = 5\ g/g.h$$

.q F demande spécifique en facteurs de croissance exprimée en g (mélange constitué à 50 % d'extrait de levure et 50 % d'un hydrolysat de caséine (caséine hydrclysée par de la trypsine) par g de cellules (poids sec) et par heure.
. q G demande spécifique en glucose exprimée en g de glucose par g de cellules (poids sec).

Pour calculer la concentration en acide lactique, on prendra un rendement de l'ordre de 80 % par rapport au glucose consommé.

A titre d'exemple en utilisant un milieu de culture dont les concentrations en glucose et en facteurs de croissance sont respectivement 63 et 31 g/l et en fixant le taux de dilution à 0,4 h$^{-1}$ (temps de rétention hydraulique de 2,5 heures), on obtient un jus de fermentation dont la fermentation en cellules est de 5 g/l (poids sec) et la concentration en acide lactique de 50 g/l. Dans ces conditions la productivité en acide lactique est de 20 g par litre de fermenteur et par heure.

REFERENCES BIBLIOGRAPHIQUES

(1) Hungate et al, 1969, p. 117-132. In J. R. Norris and D. W. Ribbons (ed.), Methods in Microbiology. Vol. 3B. Academic Press, New York.
(2) Macy et al, 1972, Amer. J. Clin. Nutr. 26:1318-1323
(3) Meshbah et al, 1989, Int. J. Syst. Bacteriol. 39:159-167.

**Revendications**

**1.** **Souche bactérienne déposée à la C.N.C.M. le 24 novembre 1993 sous le n° I-1378 et les souches de la même espèce possédant** une catalase, **ayant** une température de croissance de 25°C à 58,5°C avec une température de croissance optimale de 47°C à 53°C, **possédant** des propriétés amylolytiques et/ou capables de produire du L(+) lactate en tant que produit de fermentation.

**2.** Souches bactériennes selon la revendication 1, **caractérisées par** des conditions de croissance anaérobies et des propriétés de micro-aérophilie, avec une croissance à un pH de 5,4 à 8,5 environ.

**3.** Souches bactériennes selon la revendication 2, **caractérisées par** un optimum de croissance à un pH de 6,5 à 7,5.

**4.** Souches bactériennes selon l'une des revendications 1 à 3, **caractérisées en ce qu'**elles sont hétérolactiques et sont capables de produire à partir d'hydrates de carbone, en plus du L(+) lactate, notamment du formate, de l'acétate et de l'éthanol.

**5.** Souches bactériennes selon l'une quelconque des revendications 1 à 4, **caractérisées en ce qu'**elles sont Gram positif.

**6.** Souches bactériennes selon l'une quelconque des revendications 1 à 5, **caractérisées en ce qu'**elles se présentent sous forme de bâtonnets isolés ou en chaînes courtes avec des flagelles péritriches et sont non sporulées.

**7.** Souches bactériennes selon l'une quelconque des revendications 1 à 6, **caractérisées par** une teneur de leur ADN en guanine et cytosine inférieure à 50 mole %, notamment de l'ordre de 40 mole %.

8. Souches bactériennes selon l'une quelconque des revendications 1 à 7, **caractérisées en ce qu'**elles ne contiennent pas de cytochrome.

9. Souches bactériennes selon l'une quelconque des revendications 1 à 8, **caractérisées en ce qu'**au moins 70 % de leur génome est capable de s'hybrider avec l'ADN de la souche déposée à la C.N.C.M. le 24 novembre 1993, sous le n° I-1378.

10. Souches bactériennes selon l'une quelconque des revendications 1 à 9, **caractérisées en ce qu'**elles sont obtenues à partir de palme.

11. Procédé de culture de souches bactériennes selon l'une quelconque des revendications 1 à 10, **caractérisé en ce qu'**on opère dans des conditions anaérobies, ou tout au moins sous faible pression partielle d'oxygène, à un pH de 5,4 à 8,5 environ, en particulier de 6,5 à 7,5, dans un milieu de base contenant un sucre utilisable par ces souches comme source d'énergie et des peptides comme source d'azote.

12. Procédé selon la revendication 11, **caractérisé en ce qu'**on utilise comme source d'énergie et de carbone des hydrates de carbone, et comme source d'azote des peptides, notamment des extraits de levure ou des hydrolysats de protéines.

13. Procédé de traitement d'effluents industriels à des fins de dépollution, **caractérisé en ce qu'**il comprend la mise en oeuvre de souches bactériennes selon l'une des revendications 1 à 10.

14. Utilisation des souches bactériennes selon l'une des revendications 1 à 10 dans des procédés de fermentation alimentaire.

15. Utilisation des souches selon l'une des revendications 1 à 10 pour élaborer des mutants par transfert de matériel génétique entre ces souches, les bactéries lactiques et les bacilli.

16. Procédé de production de métabolites tels que le L(+) lactate, l'acétate, le formate, l'éthanol, **caractérisé en ce qu'**il comprend

- la culture d'une souche bactérienne selon l'une des revendications 1 à 10 dans des conditions appropriées pour son développement et pour la production du métabolite recherché,
- la récupération des métabolites produits, l'isolement du métabolite désiré et sa purification.


**Claims**

1. Bacterial strain deposited at the C.N.C.M on the 24th of November 1993 under n°I-1378 and strains of the same species having a catalase, having a growth temperature of 25°C to 58.5° C with optimal growth temperature of 47°C to 53°C, having amylolytic properties and/or capable of producing L (+)-lactate, as fermentation product.

2. Bacterial strains according to claim 1 **characterized by** anaerobic growth conditions and micro-aerophilic properties, with a growth at the pH of about 5.4 to 8.5.

3. Bacterial strains according to claim 2, **characterized by** an optimum of growth at the pH of 6.5 to 7.5.

4. Bacterial strains according to one of claims 1 to 3, **characterized in that** they are heterolactic and are capable of producing, from carbohydrates, in addition to L(+)-lactate, in particular formate, acetate and ethanol.

5. Bacterial strains according to any one claims 1 to 4, **characterized in that** they are Gram-positive.

6. Bacterial strains according to any one of claims 1 to 5, **characterized in that** they are rod-shaped, isolated or in short chains with peritrichous flagella, and are non-sporulated.

7. Bacterial strains according to any one of claims 1 to 6, **characterized by** a guanine and cytosine content of their DNA of less than 50 mol %, in particular of the order of 40 mol %.

8. Bacterial strains according to anyone of claims 1 to 7, **characterized in that** they do not contain cytochrome.

9. Bacterial strains according to anyone of claims 1 to 8 **characterized in that** at least 70% of their genome is capable of hybridizing with the DNA of the strain deposited at the C.N.C.M on the 24th of November 1993 under no.I-1378.

10. Bacterial strains according to anyone of claims 1 to 9, **characterized in that** they are obtained from palm.

11. A process for the culture of bacterial strains according to any one of claims 1 to 10, **characterized in that** it is carried out under anaerobic conditions, or at the very least under a low partial pressure of oxygen, at a pH of about 5.4 to 8.5, in particular 6.5 to 7.5, in a base medium comprising a sugar which can be utilized by these strains as a source of energy, and peptides as a source of nitrogen.

12. The process according to claim 11, **characterized in that** carbohydrates are used as a source of energy and carbon; and peptides, in particular yeast extracts or protein hydrolysates, are used as a source of nitrogen.

13. The process for the treatment of industrial effluents for the purpose of decontamination, **characterized in that** it comprises the use of bacterial strains according to one of claims 1 to 10.

14. Use of bacterial strains according to one of claims 1 to 10 in processes of food fermentation.

15. Use of the strains according to one of claims 1 to 10 to develop mutants by transfer of genetic material between these strains, lactobacteria and Bacilli.

16. A process for the production of metabolites such as L(+)-lactate, acetate, formate and ethanol, **characterized in that** comprises

- the culture of a bacterial strain according to one of claims 1 to 10 under conditions suitable for its development and for the production of the metabolite required, and
- recovery of the metabolites produced, isolation of the desired metabolite and its purification.


**Patentansprüche**

1. Bakterienstamm, der am 24. November 1993 unter der Nr. 1-1378 bei der C.N.C.M. hinterlegt wurde, und Stämme derselben Spezies, die eine Katalase besitzen, eine Wachstumstemperatur von 25 °C bis 58,5 °C mit einer optimalen Wachstumstemperatur von 47 °C bis 53 °C aufweisen, amylolytische Eigenschaften besitzen und/oder L(+)-Lactat als Fermentationsprodukt produzieren können.

2. Bakterienstämme gemäß Anspruch 1, **gekennzeichnet durch** anaerobe Wachstumsbedingungen und mikroaerophile Eigenschaften, wobei ein Wachstum bei einem pH-Wert von ungefähr 5,4 bis 8,5 erfolgt.

3. Bakterienstämme gemäß Anspruch 2, **gekennzeichnet durch** ein Wachstumsoptimum bei einem pH-Wert von 6,5 bis 7,5.

4. Bakterienstämme gemäß einem der Ansprüche 1 bis 3, **dadurch gekennzeichnet, dass** sie zur heterofermentativen Milchsäuregärung befähigt sind und aus Kohlenhydraten neben L(+)-Lactat insbesondere Formiat, Acetat und Ethanol produzieren können.

5. Bakterienstämme gemäß einem der Ansprüche 1 bis 4, **dadurch gekennzeichnet, dass** sie Gram-positiv sind.

6. Bakterienstämme gemäß einem der Ansprüche 1 bis 5, **dadurch gekennzeichnet, dass** sie in Form von isolierten Stäbchen oder in kurzen Ketten mit peritrichen Geißeln vorliegen und keine Sporen bilden.

7. Bakterienstämme gemäß einem der Ansprüche 1 bis 6, **gekennzeichnet durch** einen Guanin- und Cytosingehalt ihrer DNA von weniger als 50 Mol-%, insbesondere in der Größenordnung von 40 Mol-%.

8. Bakterienstämme gemäß einem der Ansprüche 1 bis 7, **dadurch gekennzeichnet, dass** sie kein Cytochrom enthalten.

**9.** Bakterienstämme gemäß einem der Ansprüche 1 bis 8, **dadurch gekennzeichnet, dass** wenigstens 70% ihres Genoms mit der DNA des Stamms, der am 24. November 1993 unter der Nr. 1-1378 bei der C.N.C.M. hinterlegt wurde, hybridisieren kann.

**10.** Bakterienstämme gemäß einem der Ansprüche 1 bis 9, **dadurch gekennzeichnet, dass** sie aus Palmen erhalten wurden.

**11.** Verfahren zur Kultur von Bakterienstämmen gemäß einem der Ansprüche 1 bis 10, **dadurch gekennzeichnet, dass** man unter anaeroben Bedingungen oder wenigstens unter geringem Sauerstoffpartialdruck bei einem pH-Wert von ungefähr 5,4 bis 8,5, insbesondere 6,5 bis 7,5, in einem Grundmedium arbeitet, das einen von diesen Stämmen verwertbaren Zucker als Energiequelle und Peptide als Stickstoffquelle enthält.

**12.** Verfahren gemäß Anspruch 11, **dadurch gekennzeichnet, dass** man als Energie- und Kohlenstoffquelle Kohlenhydrate und als Stickstoffquelle Peptide, insbesondere Hefeextrakte oder Proteinhydrolysate, verwendet.

**13.** Verfahren zur Behandlung von Industrieabwässern zur Wiederaufbereitung derselben, **dadurch gekennzeichnet, dass** es die Verwendung von Bakterienstämmen gemäß einem der Ansprüche 1 bis 10 umfasst.

**14.** Verwendung der Bakterienstämme gemäß einem der Ansprüche 1 bis 10 in Verfahren zur Lebensmittelfermentation.

**15.** Verwendung der Stämme gemäß einem der Ansprüche 1 bis 10 zur Entwicklung von Mutanten durch Übertragung von genetischem Material zwischen diesen Stämmen, Milchsäurebakterien und Bacillen.

**16.** Verfahren zur Produktion von Metaboliten wie L(+)-Lactat, Acetat, Formiat, Ethanol, **dadurch gekennzeichnet, dass** es Folgendes umfasst:

- die Kultur eines Bakterienstamms gemäß einem der Ansprüche 1 bis 10 unter Bedingungen, die für seine Entwicklung und für die Produktion des gewünschten Metaboliten geeignet sind;
- Gewinnung der produzierten Metaboliten, Isolierung des gewünschten Metaboliten und Reinigung desselben.

Fig. 1

Fig. 2

Fig. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **M. OKAFOR.** *J.appl. Bact.,* 1975, vol. 28, 81-88
- **HUNGATE et al.** Methods in Microbiology. Academic Press, 1969, vol. 3B, 117-132
- **MACY et al.** *Amer. J. Clin. Nutr.,* 1972, vol. 26, 1318-1323
- **MESHBAH et al.** *Int. J. Syst. Bacteriol.,* 1989, vol. 39, 159-167